# EUROPÄISCHE PATENTANMELDUNG

(11) **EP 1 142 538 A2**
(43) Veröffentlichungstag der Anmeldung: **10.10.2001**
(21) Anmeldenummer: 01108355.7
(22) Anmeldetag: 03.04.2001
(51) Int. Cl.: A61C 5/06

(54) **Einwegbehälter für dentales Füllungsmaterial**

(30) Priorität: 07.04.2000 DE 10017475
(71) Anmelder: Degussa Dental GmbH & Co. KG, 63457 Hanau (DE)
(72) Erfinder: Both, Adam, 63454 Hanau (DE); Kläres, Ulrich, 53347 Alfter (DE); Luckau, Ralf, 63796 Kahl (DE)

(57) **Zusammenfassung**

Üblicherweise verwendete bekannte Einwegbehälter für dentales Füllungsmaterial weisen einen zylinderförmigen Behälterkörper (2) mit einer als Düse (3) gestalteten Austrittsöffnung sowie einen axial beweglichen Kolben auf. Durch Verschieben des Kolbens in Richtung der Düse (3) wird dentales Füllungsmaterial aus dem Nutzraum (1) durch den Austrittskanal (4) ausgepreßt. Die Erfindung betrifft Kolben, die auf der der Düse (3) zugewandten Seite mindestens teilweise aus verformbarem Material bestehen. Wird ein entweder als Doppelkolben (5) oder als zweiteiliger Kolben ausgeführter Kolben axial in eine definierte Endstellung in Richtung der Düse (3) verschoben, so dringt verformbares Kolbenmaterial in den Austrittskanal (4) ein und verdrängt dort befindliches dentales Füllungsmaterial. Vorzugsweise ist vorgesehen, daß das in die Düse (3) eindringende Volumen des verformbaren Materials höchstens dem Volumen des Austrittskanals (4) der Düse (3) entspricht.

## Beschreibung

Die Erfindung betrifft einen Einwegbehälter für dentales Füllungsmaterial, bestehend aus einem zylinderförmigen Behälterkörper mit einer als Düse gestalteten Austrittsöffnung sowie einem axial beweglichen Kolben, mittels dessen das dentale Füllungsmaterial aus dem Einwegbehälter durch die Düse hindurch auspreßbar ist.

Für Anwendungen in einer Zahnarztpraxis werden dentalmedizinische Produkte, wie beispielsweise lichtaushärtende Füllungsmaterialien, in spezielle Einwegbehälter abgefüllt. Solche Einwegbehälter beinhalten in der Regel eine für eine einmalige Anwendung ausreichende Menge des Füllungsmaterials, welches der Zahnarzt für die Restaurierung einer präparierten Zahnkavität benötigt. Für die Applikation des Füllungsmaterials in Zahnkavitäten wird ein dauerhaft verwendbares Instrument, ein sogenannter Applikator, eingesetzt. Der nach der Applikation entleerte Einwegbehälter mit einem geringen verbleibenden Rest an Füllungsmaterial wird nicht weiter verwendet.

Bekannte Einwegbehälter der eingangs genannten Gattung weisen üblicherweise einen zylindrischen Behälterkörper auf, dessen eines Ende in Form einer abgewinkelten, leicht konischen Düse ausgestaltet ist. Sie wird mit einer Kappe verschlossen. Das andere Ende wird durch einen axial beweglichen Kolben verschlossen. Wird mit einem geeigneten Instrument, beispielsweise einem Applikator, der Kolben in Richtung der als Düse ausgestalteten Austrittsöffnung gedrückt, so wird durch diese Düse das dentale Füllungsmaterial entleert. Die Form des Kolbens ist dabei den Ausmaßen des Nutzraums im Einwegbehälter angepaßt, so daß in der Endstellung des Kolbens das dentale Füllungsmaterial vollständig aus dem Behälterkörper verdrängt wird.

Das sich im Austrittskanal der Düse befindende dentale Füllungsmaterial im Einwegbehälter kann nicht für die Restaurierung einer präparierten Zahnkavität genutzt werden. Die Länge der Düse sowie der Durchmesser des Austrittskanals werden durch die jeweilige zahnärztliche Anwendung sowie die Viskosität des dentalen Füllungsmaterials bestimmt. Deshalb kann das Volumen des Austrittkanals nicht beliebig reduziert werden, so daß meist ein vergleichsweise großer Rest des dentalen Füllungsmaterials ungenutzt im Einwegbehälter verbleibt. Wegen üblicherweise hoher Materialkosten von dentalmedizinischen Produkten stellt dieser nicht verwertbare Rest des Füllungsmaterials einen wesentlichen ökonomischen Nachteil für solche Einwegbehälter dar.

Aufgabe der Erfindung ist es daher, einen Einwegbehälter der eingangs genannten Gattung so auszugestalten, daß der nach einer zahnärztlichen Anwendung im Einwegbehälter verbleibende, nicht verwertbare Rest des dentalen Füllungsmaterials möglichst reduziert wird. Die Gestaltung der Düse soll dabei nicht verändert werden, da deren Form durch die Viskosität des Füllungsmaterials sowie die vorgesehene dentalmedizinische Anwendung weitgehend vorgegeben ist.

Diese Aufgabe wird erfindungsgemäß dadurch gelöst, daß der Kolben auf der der Düse zugewandten Seite mindestens teilweise aus verformbaren Material besteht.

Durch Verschieben des axial beweglichen Kolbens wird zuerst das dentale Füllungsmaterial vollständig aus dem Behälterkörper ausgepreßt. Weiterer Druck auf den Kolben bewirkt, daß verformbares Kolbenmaterial zumindest teilweise in die Düse eindringt und auch dort befindliches dentales Füllungsmaterial verdrängt. Eine Endstellung des Kolbens ist entweder durch die äußere Form der aus nicht deformierbarem Material bestehenden Kolbenteile genau definiert oder durch das Erreichen der Deformierbarkeit des plastischen Kolbenteils gewährleistet. Über das Volumen des verformbaren Kolbenmaterials sowie die definierte Endstellung des Kolbens kann die zusätzlich auch aus der Düse auspressbare Menge des dentalen Füllmaterials bestimmt werden.

Gemäß einer Ausgestaltung des Erfindungsgedankens ist vorgesehen, daß der Kolben als Doppelkolben ausgeführt ist, bestehend aus einer zur Düse hin offenen, nicht deformierbaren hohlzylindrischen Kolbenhülle, deren Form den Ausmaßen des Nutzraums im Einwegbehälter angepaßt ist und an deren Rückseite sich eine Öffnung befindet, sowie einem unabhängig davon axial beweglichen, radialsymmetrischen Kolbenkern aus verformbarem Material. Im Rahmen einer Anwendung werden zunächst Kolbenhülle und Kolbenkern gleichzeitig durch Druck auf die Rückseite der Kolbenhülle bis in die definierte Endstellung der Kolbenhülle verschoben und das dentale Füllungsmaterial vollständig aus dem Behälterkörper ausgepreßt. Durch die Öffnung an der Rückseite der nicht deformierbaren Kolbenhülle hindurch kann nun weiterer Druck auf den Kolbenkern ausgeübt werden. Das verformbare Material des Kolbenkerns dringt in den Austrittskanal der Düse ein und verdrängt auch dort befindliches dentales Füllungsmaterial. Der konstruktive Mehraufwand für einen derart aus mehreren Einzelteilen bestehenden Kolben ist gering verglichen mit der Einsparung an üblicherweise teurem dentalen Füllungsmaterial.

Vorzugsweise ist vorgesehen, daß sich zusätzlich zum Kolbenkern aus verformbarem Material ein der Öffnung in der Kolbenhülle zugewandter, nicht deformierbarer zylindrischer Stempel in der hohlzylindrischen Kolbenhülle befindet. Damit genügt es, durch eine vergleichsweise kleine Öffnung in der Rückseite der Kolbenhülle Druck auf den nicht verformbaren Stempel auszuüben, der von diesem gleichmäßig auf den verformbaren Kolbenkern übertragen wird. Der notwendige externe Druck kann beispielsweise durch eine auf die Rückseite der Kolbenhülle wirkende konzentrische Druckhülse sowie eine durch die Öffnung in der Kolbenrückseite auf den Stempel wirkende Druckstange erreicht werden. Druckstange und Druckhülse sind zweckmäßigerweise Bauteile eines an solche Doppelkolben angepaßten Applikators.

Entsprechend einer weiteren erfindungsgemäßen Ausführung ist es möglich, daß die aus verformbarem Material bestehende, der Düse zugewandten Seite eines zweigeteilten Kolbens in ihrer ursprünglichen Formgebung an den den Nutzraum umschließenden Behälterkörper angepaßt ist. Der Kolben besteht demzufolge aus einem nicht deformierbaren Stempel sowie einer Kolbenspitze aus verformbarem Material. Wird der Kolbenstempel axial in die durch seine Form vorgegebene Endstellung innerhalb des Behälterkörpers verschoben, so dringt ein Teil des verformbaren Materials der Kolbenspitze in die Düse ein und verdrängt dort befindliches dentales Füllungsmaterial. Da ein mit solch verformbaren Kolben versehener Einwegbehälter sich äußerlich von einem bereits handelsüblichen, mit einteiligem Kolben versehenen Einwegbehälter nicht unterscheidet, kann ein in einer Arztpraxis bereits vorhandener Applikator unverändert weiterhin angewendet werden.

Gemäß einer vorteilhaften Ausgestaltung des Erfindungsgedankens ist vorgesehen, daß das während des Verschiebens des Kolbens in die Düse eindringende Volumen des verformbaren Materials des Kolbens bei dessen Endstellung in Richtung der Düse höchstens dem Volumen des Austrittskanals der Düse entspricht. Dadurch ist prinzipiell gewährleistet, daß unter keinen Umständen verformbares Kolbenmaterial aus der Düse austritt und während einer dentalmedizinischen Anwendung in die präparierte Zahnkavität gelangt. Dies kann für beide Kolbenausführungen durch eine entsprechende Formgebung der nicht deformierbaren Kolbenteile erreicht werden.

Nachfolgend werden Ausführungsbeispiele der Erfindung näher erläutert, welche in der Zeichnung dargestellt sind. Es zeigt:

Fig. 1 einen Schnitt durch einen Einwegbehälter für dentales Füllungsmaterial, bestehend aus einem Behälterkörper mit in Form einer Düse gestalteter Austrittsöffnung sowie einem axial beweglichen Kolben.

Fig. 2 einen Schnitt durch den als Doppelkolben ausgeführten Kolben, bestehend aus einer aus nicht deformierbarem Material ausgeführten, hohlzylindrischen Kolbenhülle mit einer Öffnung an der Rückseite, in welcher sich ein ebenfalls nicht deformierbarer, rotationssymmetrischer Stempel sowie ein ebenfalls rotationssymmetrischer Kolbenkern aus verformbarem Material befinden und

Fig. 3 einen zweiteiligen Kolben, bestehend aus einem Kolbenstempel aus nicht deformierbarem Material sowie einer verformbaren Kolbenspitze.

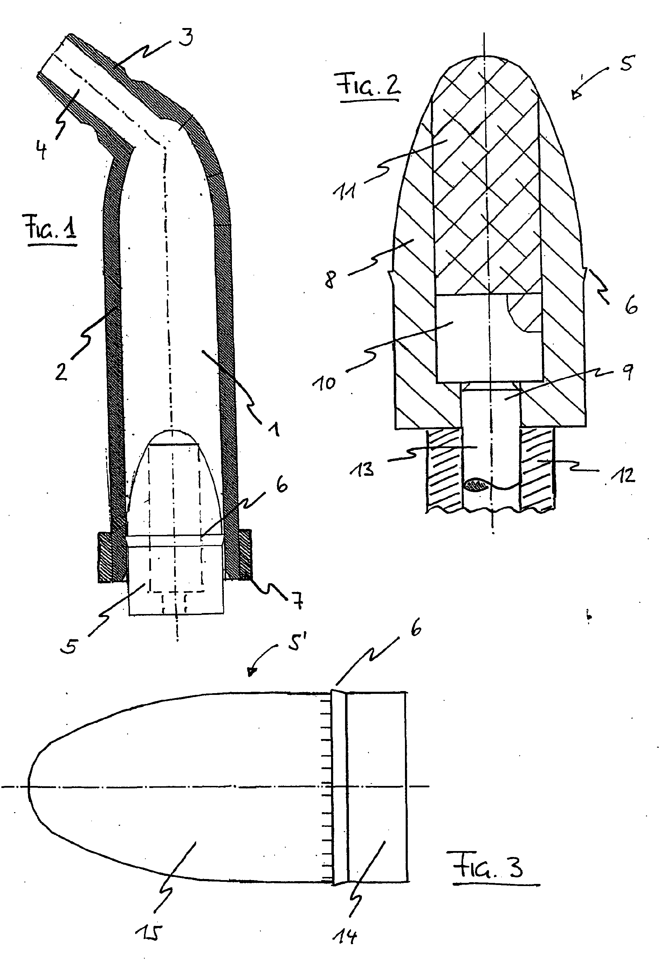

Der in Fig. 1 dargestellte Einwegbehälter weist einen den Nutzraum 1 umgebenden zylinderförmigen Behälterkörper 2 auf. An einem Ende des Behälterkörpers 2 befindet sich eine relativ zur Zylinderachse abgewinkelt ausgerichtete Düse 3. Das sich im Nutzraum 1 des Behälterkörpers 2 befindende dentale Füllungsmaterial ist durch einen Austrittskanal 4 hindurch auspressbar. Der dafür notwendige Druck wird durch einen axial im Behälterkörper 2 beweglichen Doppelkolben 5 aufgebracht. Eine sich am Doppelkolben 5 befindende radial vorspringende Dichtlippe 6 verhindert den ungewollten Austritt von dentalem Füllungsmaterial an der kolbenseitigen Öffnung 2a des Behälterkörpers 2. Während des Befüllens kann trotzdem aufgrund eines Entlüftungsprofils der Dichtlippe 6 ein notwendiger Austritt von Luft aus dem Nutzraum 1 des Behälterkörpers 2 erfolgen. Ein radial vorspringender Rand 7 am kolbenseitigen Ende des Behälterkörpers 2 dient einer besseren Druckfestigkeit des Behälters. Außerdem ergeben sich dadurch fertigungstechnische Erleichterungen bei der Positionierung des Behälters während der Befüllung in einer Abfüllanlage sowie während der Entleerung mit einem geeigneten Instrument, beispielsweise einem Applikator, im Rahmen einer dentalmedizinischen Anwendung.

Das in Fig. 2 gezeigte Ausführungsbeispiel zeigt einen Doppelkolben 5 mit einer zur Düse hin offenen, nicht deformierbaren hohlzylindrischen Kolbenhülle 8, an deren Rückseite sich eine Öffnung 9 befindet. Durch diese Öffnung 9 hindurch kann ein nicht deformierbarer Stempel 10 relativ zur Kolbenhülle 8 verschoben werden, was eine entsprechende Verschiebung des aus verformbarem Material bestehenden Kolbenkerns 11 bewirkt. Im Verlauf einer zahnmedizinischen Anwendung wird zunächst der ganze Doppelkolben 5 mittels einer konzentrischen Druckhülse 12 axial durch den Behälterkörper 2 in eine definierte Endstellung verschoben. Danach verschiebt eine Druckstange 13 den Stempel 10 im Inneren der Kolbenhülse 8 und drückt dadurch den aus verformbarem Material bestehenden Kolbenkern 11 teilweise in den Austrittskanal 4 der Düse 3, so daß dort befindliches dentales Füllungsmaterial ausgepreßt wird.

Das in Fig. 3 dargestellte Ausführungsbeispiel zeigt einen zweigeteilten Kolben 5', bestehend aus einem nicht deformierbaren mit einer Dichtlippe 6 versehenen Kolbenstempel 14 sowie einer der Düse 3 zugewandten Kolbenspitze 15, welche aus verformbarem Material besteht. Die ursprüngliche äußere Form der Kolbenspitze 15 ist den Ausmaßen des Nutzraums 1 im Behälterkörper 2 angepaßt.

## Patentansprüche

1. Einwegbehälter für dentales Füllungsmaterial, bestehend aus einem zylinderförmigen Behälterkörper mit einer als Düse gestalteten Austrittsöffnung sowie einem axial beweglichen Kolben, mittels dessen das dentale Füllungsmaterial aus dem Einwegbehälter durch die Düse hindurch auspressbar ist, **dadurch gekennzeichnet, daß** der Kolben (5, 5') auf der der Düse (3) zugewandten Seite mindestens teilweise aus verformbarem Material besteht.

2. Einwegbehälter nach Anspruch 1,
**dadurch gekennzeichnet,**
**daß** der Kolben (5) als Doppelkolben ausgeführt ist, bestehend aus einer zur Düse (3) hin offenen, nicht deformierbaren hohlzylindrischen Kolbenhülle (8), deren Form den Ausmaßen des Nutzraums (1) im Behälterkörper (2) angepaßt ist und an deren Rückseite sich eine Öffnung (9) befindet, sowie einem unabhängig davon axial beweglichen, radialsymmetrischen Kolbenkern (11) aus verformbarem Material.

3. Einwegbehälter nach Anspruch 2,
**dadurch gekennzeichnet,**
**daß** sich zusätzlich zum Kolbenkern (11) aus verformbarem Material ein der Öffnung (9) in der Kolbenhülle (8) zugewandter, nicht deformierbarer zylindrischer Stempel (10) in der hohlzylindrischen Kolbenhülle (8) befindet.

4. Einwegbehälter nach Anspruch 1,
**dadurch gekennzeichnet,**
**daß** die aus verformbarem Material bestehende, der Düse (3) zugewandten Seite eines zweigeteilten Kolbens (5') in ihrer ursprünglichen Formgebung an den den Nutzraum (1) umschließenden Behälterkörper (2) angepaßt ist.

5. Einwegbehälter nach Anspruch 1 oder 4,
**dadurch gekennzeichnet,**
**daß** das während des Verschiebens des Kolbens (5, 5') in die Düse (3) eindringende Volumen des verformbaren Materials des Kolbens (5, 5') bei dessen Endstellung in Richtung der Düse (3) höchstens dem Volumen des Austrittskanals (4) der Düse (3) entspricht.
